(19) Europäisches Patentamt

European Patent Office

Office européen des brevets

(11) EP 1 645 639 A2

(12) EUROPEAN PATENT APPLICATION

(43) Date of publication:
12.04.2006 Bulletin 2006/15

(51) Int Cl.:
*C12Q 1/68* (2006.01)

(21) Application number: 05256066.1

(22) Date of filing: 29.09.2005

(84) Designated Contracting States:
AT BE BG CH CY CZ DE DK EE ES FI FR GB GR
HU IE IS IT LI LT LU LV MC NL PL PT RO SE SI
SK TR
Designated Extension States:
AL BA HR MK YU

(30) Priority: 01.10.2004 US 957062

(71) Applicant: Agilent Technologies, Inc.
Palo Alto, CA 94306 (US)

(72) Inventors:
• Sana, Theodore R.
San Mateo,
California 94402 (US)
• Wolber, Paul K.
Los Altos,
California 94022 (US)

(74) Representative: Tollett, Ian et al
Williams Powell
Morley House
26-30 Holborn Viaduct
London EC1A 2BP (GB)

(54) **Multiple array substrates containing control probes**

(57) Multiple array substrates and methods for their use are provided. A feature of aspects of the invention is that the arrays of the multiple array substrate include a control set of probe nucleic acids that provides for the opportunity to screen for at least one of cross contamination among arrays and a probe synthesis error. Also provided are kits for practicing various aspects of the invention.

FIG. 1

**Description**

The present invention relates to multiple array substrates and methods for using the same.

Array assays between surface-bound binding agents, or probes, and target molecules in solution may be used to detect the presence of particular biopolymeric analytes in the solution. The surface-bound probes may be oligonucleotides, peptides, polypeptides, proteins, antibodies or other molecules capable of binding with target biomolecules in the solution. Such binding interactions are the basis for many of the methods and devices used in many different fields, e.g., genomics (in sequencing by hybridization, SNP detection, differential gene expression analysis, identification of novel genes, gene mapping, finger printing, etc.) and proteomics.

In use, an array surface is contacted with a labeled sample containing target analytes (usually nucleic acids or proteins) under conditions that promote specific, high-affinity binding of the analytes in the sample to one or more of the probes present on the array. The goal of this procedure is to quantify the level of binding of one or more probes of the array to labeled analytes in the sample. In some applications, the analytes in the sample are labeled with a detectable label such as a fluorescent tag, and quantification of the level of fluorescence associated with a bound probe represents a direct measurement of the level of binding. In turn, this measurement of binding represents an estimate of the abundance of a particular analyte in the sample.

As the field of array technology has progressed, multiple array substrates that contain sectors (i.e., sub-arrays) that may be independently contacted with a sample have been developed. These sectors, by virtue of design or by as a consequence of the method by which an array is made, are usually spatially separated from each other, and may be each separately contacted with a different sample in a single hybridization experiment. Such multi-sector arrays are of use in diagnostic and drug screening applications where one or more samples are incubated with multiple sets of probes, where each set of probes is contained in isolated sectors on the same substrate. Publications of interest include published U.S. Patent Application Nos. 20040126766; 20040086869; 20030231989; 20030231985; and 20030040011; and well as U.S. Patent No. 5,874,219.

Because of the potential utility of multiple array substrates in a variety of different applications, there is continued interest in the development of new features for this array format.

According to a first aspect of the present invention there is provided a multiple array substrate as claimed in claim 1.

According to a second aspect of the present invention there is provided a method as claimed in claim 9.

According to a third aspect of the present invention there is provided a kit as claimed in claim 13.

Multiple array substrates and methods for their use are provided. A feature of aspects of the invention is that the arrays of the multiple array substrates include a control set of probe nucleic acids that provides for the opportunity to screen for at least one of cross contamination among arrays and a probe synthesis error. Also provided are kits for practicing various aspects of the invention.

Aspects of the subject invention provide methods of determining at least one of a probe synthesis error and cross-contamination in an assay employing an array. The methods of these aspects may include contacting: (i) a first sample with a first sub-array of a multiple array substrate, wherein said first sample comprises a first control target nucleic acid; and (ii) a second sample with a second sub-array of the multiple array substrate. In these embodiments, each of said first and second sub-arrays includes a set of at least four control probe nucleic acids each having at least a first probe domain of from about 4 to about 30 nucleic acid residues, wherein each member probe nucleic acid of the set has a different base from any other member of the set at each residue position of said first probe domain. Following this contacting step, the methods include detecting binding of the first control target nucleic acid to the first set of control probes in each of the first and second sub-arrays, e.g., to screen for the presence of at least one of, and sometimes both of: a probe synthesis error; and cross-contamination. In certain embodiments, the multiple array substrate includes at least four, and sometimes at least eight, sub-arrays, each including the set of at least four control probes. In certain embodiments, each member probe nucleic acid of the set further includes a second probe domain of about 4 to about 30 nucleotide residues, wherein each member probe nucleic acid of the set has a different base from any other member of the set at each residue position of said second probe domain. In certain embodiments, each second probe domain of the set has a sequence that is identical to the sequence of a first probe domain of the set, where the first and second domains of a given probe are not identical. In certain embodiments, each member probe nucleic acid of the set further includes a tether domain between the first probe domain and the substrate. In certain embodiments, the set of at least four control probe nucleic acids each have the structure:

$$T\text{-}D_1\text{-}D_2$$

where:

T is an optional tether sequence;
D1 is a first probe domain; and
D2 is a second probe domain.

In certain embodiments, each first probe domain D1 has a sequence chosen from the group consisting of A, B, C and D, wherein each of A, B, C and D has a unique sequence of from 4 to 30 nt in length. In certain embodiments, each second probe domain D2 has a sequence chosen from the group consisting of A, B, C and D. In certain embodiments, the set of at least four control probe nucleic acids is described by the formula:

$$T\text{-}A\text{-}B$$

$$T\text{-}B\text{-}A$$

$$T\text{-}C\text{-}D$$

$$T\text{-}D\text{-}C$$

where A, B, C and D may have the following formulas:

$$A = GGTCAGTGTATCCAGTTTCTCCGAA \text{ (SEQ ID NO: 05)};$$

$$B = ATCATCGTAGCTGGTCAGTGTATCC \text{ (SEQ ID NO: 06)};$$

$$C = TCAGGTCAGTGATTCACCGAATCTG \text{ (SEQ ID NO: 07)};$$

and

$$D = CAGTCAACCCAGACAGGAACGGAGT \text{ (SEQ ID NO: 08)}.$$

In certain embodiments, the first control target nucleic acid is complementary to a first probe domain of a first probe nucleic acid of said set. In certain embodiments, the method further includes having a second control target nucleic in the second sample, wherein the second control target nucleic acid has a sequence that is different from said first control target nucleic acid and is complementary to a first probe domain of a second probe nucleic acid of said set. In certain embodiments, the method includes transmitting a result obtained from a method from a first location to a second location, which location may be a remote location. Also provided are methods of receiving a transmitted result of a method according to the invention.

Additional aspects of the invention include a multiple array substrate that includes at least a first sub-array and a second sub-array, wherein each of the first and second sub-arrays includes a set of at least four control probe nucleic acids each having at least a first probe domain of from about 4 to about 30 nucleic acid residues, wherein each member probe nucleic acid of the set has a different base from any other member of said set at each residue position of said first probe domain, e.g., as described above.

Additional aspects of the invention further includes kits that include a multiple array substrate, e.g., as described above, and a first control target nucleic acid that is complementary to a first probe domain of a first probe nucleic acid of the set. Such kits may include a second control target nucleic that has a sequence that is different from the first control target nucleic acid and is complementary to a first probe domain of a second probe nucleic acid of the set.

Embodiments of the present invention are described below, by way of example only, with reference to the accompanying drawing in which:

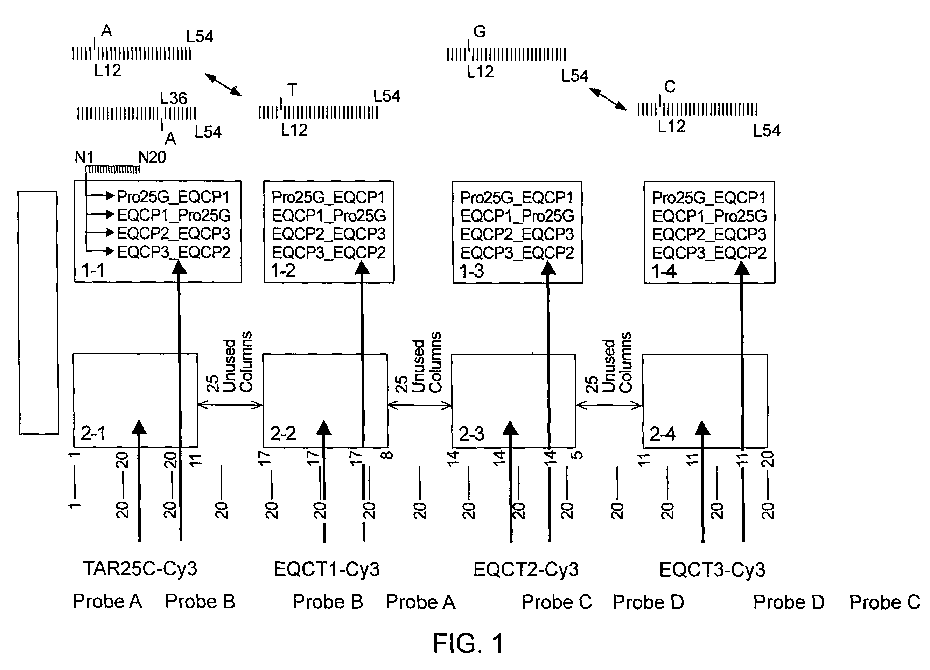

Figure 1 provides a depiction of an 8-plex multiple array substrate with each sub-array containing the same set of 4 control probes according to an embodiment of the subject invention.

Unless defined otherwise, all technical and scientific terms used herein have the same meaning as commonly understood by one of ordinary skill in the art to which this invention belongs. Still, certain elements are defined below for the sake of clarity and ease of reference.

As used herein, the term "contacting" means to bring or put together. As such, a first item is contacted with a second item when the two items are brought or put together, e.g., by touching them to each other.

The term "sample" as used herein refers to a fluid composition, where in certain embodiments the fluid composition is an aqueous composition.

The phrase "multiple array substrate", "multiple array" or "multiplex array" refers to an array configuration in which more than one distinct chemical array (which may also be referred to as a sub-array) is present on a surface of a common substrate. Multiple array substrates may take a variety of different, configurations, where representative configurations are disclosed in: U.S. Patent Application Nos. 20040126766; 20040086869; 20030231989; 20030231985; and 20030040011; and well as U.S. Patent No. 5,874,219.

The phrase "control target nucleic acid" refers to a nucleic acid that is employed in the subject methods as a target nucleic acid that hybridizes to the features of the set of control probe nucleic acids and gives rise to signals from the set of control probe nucleic acids that are employed to determine screen for at least one a probe synthesis error and cross contamination. The sequence of the control target nucleic acid is chosen so that the target specifically binds to the features of a set of control probe features. In certain aspects, the target is in a sample being assayed, and has a sequence such that it does not detectably bind to other targets in the sample or to probes other than the probes of the corresponding set of control probe features. Hybridization parameter target sequences typically have a sequence that is not present in and will not hybridize to the genome of an organism represented by the corresponding non-hybridization parameter probes on an array. In other words, if an array contains probes for genes and gene products of a specific species, e.g., humans, the hybridization parameter target sequences in a sample that is intended to be incubated with the array will have a sequence that is not represented in the genome of that species or its products. For example, in embodiments involving samples containing targets from humans, control target nucleic acids may include sequences from yeast, bacteria or any other organism, or may have any other sequence, such that they will not specifically bind to probes for human targets.

The phrase "a set of at least four control probe nucleic acids" refers to a set of at least four probe nucleic acids of differing sequence, where each control probe nucleic acid of the set includes at least a first probe domain of from about 4 to about 30 nucleotide residues, wherein each member probe nucleic acid of the set has a different base from any other member of the set at each residue position of said first probe domain. For example, at position or residue 1 of a given first probe domain, control probe 1 of the set may have A, control probe 2 of the set may have G, control probe 3 of the set may have C and control probe 4 of the set may have T. In certain embodiments, the control probe nucleic acids further include a second probe domain that is analogous to the first probe domain, and may also include a tether domain, as reviewed in greater detail below.

As used herein, the term "detecting" means to ascertain a signal, either qualitatively or quantitatively.

The term "binding" refers to two objects associating with each other to produce a stable composite structure. In certain embodiments, binding between two complementary nucleic acids may be referred to as specifically hybridizing. The terms "specifically hybridizing," "hybridizing specifically to" and "specific hybridization" and "selectively hybridize to," are used interchangeably and refer to the binding, duplexing, or hybridizing of a nucleic acid molecule preferentially to a particular nucleotide sequence under stringent conditions.

The term "screening" refers to determining the presence of something of interest, e.g., an analyte, an occurrence, etc. As used herein, the term "determining" means to identify, i.e., establishing, ascertaining, evaluating or measuring, a value for a particular parameter of interest, e.g., a hybridization parameter. The determination of the value may be qualitative (e.g., presence or absence) or quantitative, where a quantitative determination may be either relative (i.e., a value whose units are relative to a control (i.e., reference value) or absolute (e.g., where a number of actual molecules is determined).

The phrase "probe synthesis error" refers to the an error in the production of a probe nucleic acid, e.g., via *in situ* deposition methodology

"Cross-contamination" between two or more samples is an undesirable mixing of the samples. A cross-contaminated sample may contain about 1 %, about 1%-5%, about 5% to about 10%, about 10%-about 20%, about 20% to about 30%, or about 30% to about 50% or more, by volume, of another sample. Cross-contamination of samples contacted with arrays on a multi-array substrate may occur prior to or during the period of contact of the samples with the arrays (i.e., incubation of the samples and the array), however, cross-contamination of samples may also occur during washing of the arrays after the period of contact.

The term "complementary" is employed to refer to a measure or degree of pairing of complementary nucleotide bases (adenine and thymine, guanine and cytosine) to each other via hydrogen bonds from opposite strands of a double stranded nucleic acid (such as DNA or RNA). As such, complementary sequences are nucleic acid base sequences that can form a double-stranded structure, i.e., duplex, by matching base pairs. For example, the complementary sequence to G-T-A-C is C-A-T-G. Accordingly, two nucleotide sequences are "complementary" to one another when those molecules share base pair organization homology. "Complementary" nucleotide sequences will combine with specificity to form a stable duplex under appropriate hybridization conditions. For instance, two sequences are complementary when a section of a first sequence can bind to a section of a second sequence in an anti-parallel sense wherein the 3'-end of each sequence binds to the 5'-end of the other sequence and each A, T(U), G, and C of one sequence is then aligned with a T(U), A, C, and G, respectively, of the other sequence. RNA sequences can also include complementary G=U or U=G base pairs. Thus, two sequences need not have perfect homology to be "complementary" under the invention, and in most situations two sequences are sufficiently complementary when at least about 85% (preferably at least about 90%, and most preferably at least about 95%) of the nucleotides share base pair organization over a defined length of the molecule.

As used herein, the phrase "nucleic acid array hybridization assay" refers to an assay in which a nucleic acid array comprising "probe" sequences is employed. In these assays, a sample of target nucleic acids is first prepared from the initial nucleic acid sample being assayed, where preparation may include labeling of the target nucleic acids with a label, (e.g., such as a member of signal producing system, for example a fluorescent label). Following target nucleic acid sample preparation, the sample is contacted with an array comprising probe features of probe nucleic acid sequences under hybridization conditions, e.g., stringent hybridization conditions, and complexes are formed between target nucleic acids that are sufficiently complementary to probe sequences attached to the array surface. The presence of the resultant complexes is then detected, either qualitatively or quantitatively. Specific hybridization technology which may be practiced to generate the expression profiles employed in the subject methods includes, but is not limited to, the technology described in U.S. Patent Nos.: 6, 656, 740; 6, 613, 893; 6, 599, 693; 6, 589, 739; 6, 587, 579; 6, 420,180; 6,387,636; 6,309,875; 6,232,072; 6,221,653; and 6,180,351 and the references cited therein.

"Reading" signal data from an array refers to the detection of the signal data (such as by a detector) from the array. This data may be saved in a memory (whether for relatively short or longer terms).

The term "nucleic acid" includes DNA, RNA (double-stranded or single stranded), analogs (e.g., PNA or LNA molecules) and derivatives thereof. The terms "ribonucleic acid" and "RNA" as used herein mean a polymer composed of ribonucleotides. The terms "deoxyribonucleic acid" and "DNA" as used herein mean a polymer composed of deoxyribonucleotides. The term "mRNA" means messenger RNA. An "oligonucleotide" generally refers to a nucleotide multimer of about 10 to 100 nucleotides in length, while a "polynucleotide" includes a nucleotide multimer having any number of nucleotides. As such, the term "nucleic acid" includes polymers in which the conventional backbone of a polynucleotide has been replaced with a non-naturally occurring or synthetic backbone, and nucleic acids (or synthetic or naturally occurring analogs) in which one or more of the conventional bases has been replaced with a group (natural or synthetic) capable of participating in Watson-Crick type hydrogen bonding interactions. Polynucleotides include single or multiple stranded configurations, where one or more of the strands may or may not be completely aligned with another. A, "nucleotide" refers to a sub-unit of a nucleic acid and has a phosphate group, a 5 carbon sugar and a nitrogen containing base, as well as functional analogs (whether synthetic or naturally occurring) of such sub-units which in the polymer form (as a polynucleotide) can hybridize with naturally occurring polynucleotides in a sequence specific manner analogous to that of two naturally occurring polynucleotides.

The phrase "nucleic acid array" refers to an array of nucleic acid features. An "array," includes any one-dimensional, two-dimensional or substantially two-dimensional (as well as a three-dimensional) arrangement of addressable regions bearing a particular nucleic acid moiety or moieties (e.g., polynucleotide or oligonucleotide sequences, etc.) associated with that region. The nucleic acids may be covalently attached to the arrays at any point along the nucleic acid chain, but are generally attached at one of their termini (e.g. the 3' or 5' terminus).

Any given substrate may carry one, two, four or more or more arrays disposed on a front surface of the substrate. Depending upon the use, any or all of the arrays may be the same or different from one another and each may contain multiple spots (also referred to herein as "features"). A typical array may contain more than ten, more than one hundred, more than one thousand more than ten thousand features, or even more than one hundred thousand features, in an area of less than 20 cm$^2$ or even less than 10 cm$^2$. For example, features may have widths (that is, diameter, for a round spot) in the range of from about 10 $\mu$m to about 1.0 cm. In other embodiments each feature may have a width in the range of about 1.0 $\mu$m to about 1.0 mm, such as from about 5.0 $\mu$m to about 500 $\mu$m, and including from about 10 $\mu$m to about 200 $\mu$m. Non-round features may have area ranges equivalent to that of circular features with the foregoing width (diameter) ranges. A given feature is made up of nucleic acids that hybridize to the same target nucleic acid, such that a given feature corresponds to a particular target nucleic acid. At least some, or all, of the features are of different compositions (for example, when any repeats of each feature composition are excluded the remaining features may account for at least 5%, 10%, or 20% of the total number of features). Interfeature areas will typically (but not essentially) be present which do not carry any polynucleotide. Such interfeature areas typically will be present where the arrays are formed by processes involving drop deposition of reagents but may not be present when, for example, light directed synthesis fabrication processes are used. It will be appreciated though, that the interfeature areas, when present, could be of various sizes and configurations.

Each array may cover an area of less than 100 cm$^2$, or even less than 50 cm$^2$, 10 cm$^2$ or 1 cm$^2$. In certain embodiments, the substrate carrying the one or more arrays will be shaped generally as a rectangular solid (although other shapes are possible), having a length of more than 4 mm and less than 1 m, usually more than 4 mm and less than 600 mm, more usually less than 400 mm; a width of more than 4 mm and less than 1 m, usually less than 500 mm and more usually less than 400 mm; and a thickness of more than 0.01 mm and less than 5.0 mm, usually more than 0.1 mm and less than 2 mm and more usually more than 0.2 and less than 1 mm. With arrays that are read by detecting fluorescence, the substrate may be of a material that emits low fluorescence upon illumination with the excitation light. Additionally in this situation, the substrate may be relatively transparent to reduce the absorption of the incident illuminating laser light and subsequent heating if the focused laser beam travels too slowly over a region. For example, substrate may transmit at least 20%, or 50% (or even at least 70%, 90%, or 95%), of the illuminating light incident on the front as may be measured across the entire integrated spectrum of such illuminating light or alternatively at 532 nm or 633 nm.

Arrays can be fabricated using drop deposition from pulsejets of either polynucleotide precursor units (such as monomers) in the case of *in situ* fabrication, or the previously obtained polynucleotide. Such methods are described in detail in, for example, the previously cited references including US 6,242,266, US 6,232,072, US 6,180,351, US 6,171,797, US 6,323,043, U.S. Patent Application Serial No. 09/302,898 filed April 30, 1999 by Caren et al., and the references cited therein. Other drop deposition methods can be used for fabrication, as previously described herein. Also, instead of drop deposition methods, light directed fabrication methods may be used, as are known in the art. Interfeature areas need not be present particularly when the arrays are made by light directed synthesis protocols.

An array is "addressable" when it has multiple regions of different moieties (e.g., different polynucleotide sequences) such that a region (i.e., a "feature" or "spot" of the array) at a particular predetermined location (i.e., an "address") on the array will detect a particular target or class of targets (although a feature may incidentally detect non-targets of that feature). Array features are typically, but need not be, separated by intervening spaces. In the case of an array, the "target" will be referenced as a moiety in a mobile phase (typically fluid), to be detected by probes ("target probes") which are bound to the substrate at the various regions. However, either of the "target" or "target probe" may be the one which is to be evaluated by the other (thus, either one could be an unknown mixture of polynucleotides to be evaluated by binding with the other). A "scan region" refers to a contiguous (preferably, rectangular) area in which the array spots or features of interest, as defined above, are found. The scan region is that portion of the total area illuminated from which the resulting fluorescence is detected and recorded. For the purposes of this invention, the scan region includes the entire area of the slide scanned in each pass of the lens, between the first feature of interest, and the last feature of interest, even if there exist intervening areas which lack features of interest. An "array layout" refers to one or more characteristics of the features, such as feature positioning on the substrate, one or more feature dimensions, and an indication of a moiety at a given location. "Hybridizing" and "binding", with respect to polynucleotides, are used interchangeably.

The term "substrate" as used herein refers to a surface upon which marker molecules or probes, e.g., an array, may be adhered. Glass slides are the most common substrate for biochips, although fused silica, silicon, plastic and other materials are also suitable.

The term "flexible" is used herein to refer to a structure, e.g., a bottom surface or a cover, that is capable of being bent, folded or similarly manipulated without breakage. For example, a cover is flexible if it is capable of being peeled away from the bottom surface without breakage.

"Flexible" with reference to a substrate or substrate web, references that the substrate can be bent 180 degrees around a roller of less than 1.25 cm in radius. The substrate can be so bent and straightened repeatedly in either direction at least 100 times without failure (for example, cracking) or plastic deformation. This bending must be within the elastic limits of the material. The foregoing test for flexibility is performed at a temperature of 20 °C.

A "web" references a long continuous piece of substrate material having a length greater than a width. For example, the web length to width ratio may be at least 5/1, 10/1, 50/1, 100/1, 200/1, or 500/1, or even at least 1000/1.

The substrate may be flexible (such as a flexible web). When the substrate is flexible, it may be of various lengths including at least 1 m, at least 2 m, or at least 5 m (or even at least 10 m).

The term "rigid" is used herein to refer to a structure e.g., a bottom surface or a cover that does not readily bend without breakage, i.e., the structure is not flexible.

A signal refers to any detectable (i.e., identifiable) indicator of the presence or occurrence of an event of interest, e.g., a binding event between two complementary nucleic acids. Signals that are detected in the present invention may vary depending on the signal producing system employed, where the signals may be isotopic, fluorescent, electrical, etc., where in representative embodiments the signals of interest are fluorescent emissions, as is known in the art. The signals observed in the methods of the subject invention are, in certain aspects, generated by a signal producing system. As is known in the art, signal producing systems may vary with respect to the nature of the label system employed therein. Labels of interest include directly detectable and indirectly detectable radioactive or non-radioactive labels such as fluorescent dyes. Directly detectable labels are those labels that provide a directly detectable signal without interaction with one or more additional chemical agents. Examples of directly detectable labels include fluorescent labels. Indirectly detectable labels are those labels which interact with one or more additional members to provide a detectable signal. In this latter embodiment, the label is a member of a signal producing system that includes two or more chemical agents that work together to provide the detectable signal. Examples of indirectly detectable labels include biotin or digoxigenin, which, can be detected by a suitable antibody coupled to a fluorochrome or enzyme, such as alkaline phosphatase. In many preferred embodiments, the label is a directly detectable label. Directly detectable labels of particular interest include fluorescent labels. Fluorescent labels that find use in the subject invention include a fluorophore moiety. Specific fluorescent dyes of interest include: xanthene dyes, e.g., fluorescein and rhodamine dyes, such as fluorescein isothiocyanate (FITC), 2-[ethylamino)-3-(ethylimino)-2-7-dimethyl-3H-xanthen-9-yl] benzoic acid ethyl ester monohydrochloride (R6G)(emits a response radiation in the wavelength that ranges from about 500 to 560 nm), 1, 1, 3, 3, 3', 3'-Hexamethylindodicarbocyanine iodide (HIDC) (emits a response radiation in the wavelength that ranged from about 600 to 660 nm), 6-carboxyfluorescein (commonly known by the abbreviations FAM and F),6-carboxy-2',4',7',4,7-hexachlorofluorescein (HEX), 6-carboxy-4', 5'-dichloro-2', 7'-dimethoxyfluorescein (JOE or J), N,N,N',N'-tetramethyl-6-carboxyrhodamine (TAMRA or T), 6-carboxy-X-rhodamine (ROX or R), 5-carboxyrhodamine-6G (R6G5 or G5), 6-carboxyrhodamine-6G (R6G6 or G6), and rhodamine 110; cyanine dyes, e.g. Cy3, Cy5 and Cy7 dyes; coumarins, e.g., umbelliferone; benzimide dyes, e.g. Hoechst 33258; phenanthridine dyes, e.g. Texas Red; ethidium dyes; acridine dyes; carbazole dyes; phenoxazine dyes; porphyrin dyes; polymethine dyes, e.g. cyanine dyes such as Cy3 (emits a response radiation in the wavelength that ranges from about 540 to 580 nm), Cy5 (emits a response radiation in the wavelength that ranges from about 640 to 680 nm), etc; BODIPY dyes and quinoline dyes. Specific fluorophores of interest include: Pyrene, Coumarin, Diethylaminocoumarin, FAM, Fluorescein Chlorotriazinyl, Fluorescein, R110, Eosin, JOE, R6G, HIDC, Tetramethylrhodamine, TAMRA, Lissamine, ROX, Napthofluorescein, Texas Red, Napthofluorescein, Cy3, and Cy5, and the like.

The term "stringent conditions" refers to conditions under which a probe will hybridize preferentially to its target subsequence, and to a lesser extent to, or not at all to, other sequences. Put another way, the term "stringent hybridization conditions" as used herein refers to conditions that are compatible to produce duplexes on an array surface between complementary binding members, e.g., between probes and complementary targets in a sample, e.g., duplexes of nucleic acid probes, such as DNA probes, and their corresponding nucleic acid targets that are present in the sample, e.g., their corresponding mRNA analytes present in the sample. A "stringent hybridization" and "stringent hybridization wash conditions" in the context of nucleic acid hybridization (e.g., as in array, Southern or Northern hybridizations) are sequence dependent, and are different under different environmental parameters. Stringent hybridization conditions that can be used to identify nucleic acids within the scope of the invention can include, e.g., hybridization in a buffer comprising 50% formamide, 5×SSC, and 1% SDS at 42°C, or hybridization in a buffer comprising 5×SSC and 1% SDS at 65°C, both with a wash of 0.2xSSC and 0.1% SDS at 65°C. Exemplary stringent hybridization conditions can also include a hybridization in a buffer of 40% formamide, 1 M NaCl, and 1% SDS at 37°C, and a wash in 1×SSC at 45°C. Alternatively, hybridization to filter-bound DNA in 0.5 M $NaHPO_4$, 7% sodium dodecyl sulfate (SDS), 1 mnM EDTA at 65°C, and washing in 0.1xSSC/0.1 % SDS at 68°C can be employed. Yet additional stringent hybridization conditions include hybridization at 60°C or higher and 3 x SSC (450 mM sodium chloride/45 mM sodium citrate) or incubation at 42°C in a solution containing 30% formamide, 1 M NaCl, 0.5% sodium sarcosine, 50 mM MES, pH 6.5. Those of ordinary skill will readily recognize that alternative but comparable hybridization and wash conditions can be utilized to provide conditions of similar stringency.

In certain embodiments, the stringency of the wash conditions set forth the conditions which determine whether a nucleic acid is specifically hybridized to a probe. Wash conditions used to identify nucleic acids may include, e.g.: a salt concentration of about 0.02 molar at pH 7 and a temperature of at least about 50 °C or about 55°C to about 60°C; or, a salt concentration of about 0.15 M NaCl at 72°C for about 15 minutes; or, a salt concentration of about 0.2xSSC at a temperature of at least about 50°C or about 55 °C to about 60°C for about 15 to about 20 minutes; or, the hybridization complex is washed twice with a solution with a salt concentration of about 2xSSC containing 0.1 % SDS at room temperature for 15 minutes and then washed twice by 0.1×SSC containing 0.1 % SDS at 68°C for 15 minutes; or, equivalent conditions. Stringent conditions for washing can also be, e.g., 0.2xSSC/0.1% SDS at 42°C. In instances wherein the nucleic acid molecules are deoxyoligonucleotides ("oligos"), stringent conditions can include washing in 6×SSC/0.05% sodium pyrophosphate at 37 °C (for 14-base oligos), 48 °C (for 17-base oligos), 55°C (for 20-base oligos), and 60°C (for 23-base oligos). See Sambrook et al., Molecular Cloning: A Laboratory Manual (2001) for detailed descriptions of equivalent hybridization and wash conditions and for reagents and buffers, e.g., SSC buffers and equivalent reagents and conditions.

Stringent hybridization conditions are hybridization conditions that are at least as stringent as the above representative conditions, where conditions are considered to be at least as stringent if they are at least about 80% as stringent, typically at least about 90% as stringent as the above specific stringent conditions. Other stringent hybridization conditions are known in the art and may also be employed, as appropriate.

The terms "reference" and "control" are used herein interchangeably to refer to a set of values against which a set of experimentally obtained values may be compared to determine a hybridization pattern of interest. The reference can be in the form of a standardized pattern, e.g., of signals from features obtained under varying values of the hybridization pattern of interest. For example, the reference may be a standardized pattern of signals obtained from a set of hybridization parameter probe features under a series of different experiments in which all but the temperature is held constant, such that one has set of signals in the pattern that are obtained at a plurality of different temperatures. Similarly, the reference may be a standardize pattern of signals obtained from a set of hybridization parameter probe features under a series of different experiments in which all but the salt concentration is held constant, such that one has set of signals in the pattern that are obtained at a plurality of different salt concentrations.

By "remote location," it is meant a location other than the location at which the array is present and hybridization occurs. For example, a remote location could be another location (e.g., office, lab, etc.) in the same city, another location in a different city, another location in a different state, another location in a different country, etc. As such, when one item is indicated as being "remote" from another, what is meant is that the two items are at least in different rooms or different buildings, and may be at least one mile, ten miles, or at least one hundred miles apart.

"Communicating" information references transmitting the data representing that information as electrical signals over a suitable communication channel (e.g., a private or public network). "Forwarding" an item refers to any means of getting that item from one location to the next, whether by physically transporting that item or otherwise (where that is possible) and includes, at least in the case of data, physically transporting a medium carrying the data or communicating the data.

A "computer-based system" refers to the hardware means, software means, and data storage means used to analyze the information of the present invention. The minimum hardware of the computer-based systems of the present invention comprises a central processing unit (CPU), input means, output means, and data storage means. A skilled artisan can readily appreciate that any one of the currently available computer-based system are suitable for use in the present invention. The data storage means may comprise any manufacture comprising a recording of the present information as described above, or a memory access means that can access such a manufacture.

To "record" data, programming or other information on a computer readable medium refers to a process for storing information, using any such methods as known in the art. Any convenient data storage structure may be chosen, based on the means used to access the stored information. A variety of data processor programs and formats can be used for storage, e.g. word processing text file, database format, etc.

A "processor" references any hardware and/or software combination that will perform the functions required of it. For example, any processor herein may . be a programmable digital microprocessor such as available in the form of a electronic controller, mainframe, server or personal computer (desktop or portable). Where the processor is programmable, suitable programming can be communicated from a remote location to the processor, or previously saved in a computer program product (such as a portable or fixed computer readable storage medium, whether magnetic, optical or solid state device based). For example, a magnetic medium or optical disk may carry the programming, and can be read by a suitable reader communicating with each processor at its corresponding station.

Multiple array substrates and methods for their use are provided. A feature of aspects of the invention is that the arrays of the multiple array substrates' include a control set of probe nucleic acids that provides for the opportunity to screen for at least one of cross contamination among arrays and a probe synthesis error. Also provided are kits for practicing various aspects of the invention.

Before the subject invention is described further, it is to be understood that the invention is not limited to the particular embodiments of the invention described below, as variations of the particular embodiments may be made and still fall within the scope of the appended claims. It is also to be understood that the terminology employed is for the purpose of describing particular embodiments, and is not intended to be limiting. Instead, the scope of the present invention will be established by the appended claims.

In this specification and the appended claims, the singular forms "a," "an" and "the" include plural reference unless th context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

Where a range of values is provided, it is understood that each intervening value, to the tenth of the unit of the lower limit unless the context clearly dictates otherwise, between the upper and lower limit of that range, and any other stated or intervening value in that stated range, is encompassed within the invention., The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood to one of ordinary skill in the art to which this invention belongs. Although any methods, devices and materials similar or equivalent to those described herein can be used in the practice or testing of the invention, the preferred methods, devices and materials are now described. Methods recited herein may be carried out in any order of the recited events which is logically possible, as well as the recited order of events.

All patents and other references cited in this application, are incorporated into this application by reference except insofar as they may conflict with those of the present application (in which case the present application prevails). The citation of any publication is for its disclosure prior to the filing date and should not be construed as an admission that the present invention is not entitled to antedate such publication by virtue of prior invention.

METHOD COMPONENTS

*Arrays*

One aspect of the invention includes nucleic acid arrays (i.e., multiple array substrates) containing at least a first array and a second array (also referred to herein as "sub-arrays") that can be contacted to independent samples simultaneously. In certain embodiments, these multiple-array substrates can have more than 2 sub-arrays, including 4 sub-arrays, 8 sub-arrays, or 12 sub-arrays or more, e.g., 48, 96, or 384 or more sub-arrays.

The number of distinct nucleic acid sequences, and hence spots or similar structures (i.e., array features), present on any sub-array of the multiple-array may vary, but is generally at least 2, usually at least 5 and more usually at least 10, where the number of different spots on a given sub-array may be as a high as 50, 100, 500, 1000, 10,000 or higher, depending on the intended use of the array. The spots of distinct nucleic acids present on each sub-array surface are generally present as a pattern, where the pattern may be in the form of organized rows and columns of spots, e.g., a grid of spots, across the substrate surface, a series of curvilinear rows across the substrate surface, e.g., a series of concentric circles or semi-circles of spots, and the like. The density of spots present on the surface of each array may vary, and may be at least about 10 spots/cm$^2$, such as at least about 100 spots/cm$^2$, where the density may be as high as $10^6$ spots/cm$^2$or, higher, and in some embodiments does not exceed about $10^5$ spots/cm$^2$. The nucleic acids may be covalently attached to the arrays at any point along the nucleic acid chain, but are generally attached at one of their termini, e.g., the 3' or 5' terminus. In certain embodiments, the nucleic acid probes bound to the substrate present in each array of the multiple array substrate are synthesized directly on the substrate, such that the arrays may be called *in situ* fabricated arrays.

*Probes*

A novel feature of the subject multiple-arrays is that they include within the totality of nucleic acid probes on the surface of the array a collection of control probes which allows for the detection of at least one of: 1) probe synthesis errors that occurred during the fabrication process; and 2) cross-contamination between samples contacted to each sub-array of the multiple-array. The control probe sets within the collection of control probes that find use in the subject invention are "all-bases-all-layers" control probe sets, where such sets are described in United States patent application serial no. 10/407,090. A given set of "all-bases-all-layers" control probes is made up of at least four different control probe nucleic acids of differing sequence each having at least a first probe domain of from about 4 to about 30 nucleotide residues, where each member probe nucleic acid of the set has a different base from any other member of the set at each residue position of the first probe domain. In the example below, at position or residue 1 of a given probe domain, control probe 1 of the set may have A, control probe 2 of the set may have C, control probe 3 of the set may have T and control probe 4 of the set may have G. An exemplary set of four 6-mer control probes would be:

> 5'-ATGCTC-surface    Probe 1
> 5'-CACTGA-surface    Probe 2
> 5'-TGAGCT-surface    Probe 3
> 5'-GCTAAG-surface    probe 4

where each probe of the set has a single probe domain of 6 residues long. Regardless of their domain structure, control probes typically do not contain appreciable secondary structure under suitable hybridization conditions which may interfere with specific target binding.

A given collection of "all-bases-all-layers" control probes may be made up of a single set of control probes or a plurality of different sets of control probes. In general, the number of control probe sets in the collection of control probes used depends on many factors, including the length of test probes in a given multiple-array and the number of sub-arrays present on the multiple-array. Thus, in certain embodiments, a collection of "all-bases-all-layers" control probes according to the subject invention may be made up of a single set of control probes. In yet other embodiments, a collection of "all-bases-all-layers" control probes according to the subject invention may be made up of a plurality of different sets of control probes,
where plurality means at least 2, wherein in certain embodiments, the number of sets is more than 2, for example 3, 4, or 5 or more sets.

In certain embodiments, when arranging the collection of control probes on the multiple array substrate, each sub-array contains at least one set of control probes. In some embodiments, the set of control probes present on each sub-array of a multiple-array is identical. In other words, if 4 distinct control probes are in a given set for one sub-array of a multiple-array, the same control probe set will be present on all of the other sub-arrays of the multiple array. In some embodiments, different sub-arrays may contain different control probe sets. As such, the number of control probe sets in the collection of control probes used on a multiple-array substrate of the subject invention can vary. Furthermore, some or all of the sub-arrays of a multiple-array can contain more than one control probe set. Within each sub-array, each control probe set may be present in duplicate, triplicate, quadruplicate, etc., as needed.

As mentioned above, the control probes of a given set of control probes include at least one probe domain of from about 4 to about 30 nt in length, where the length may range from about 5 to about 30 nt in length, e.g., about 10 to about 30 nt in length, about 15 to about 25 nt in length, etc. In certain embodiments, the control probes that make up a given set include a single probe domain. In yet other embodiments, the control probes include a plurality of two or more probe domains, where the number of different probe domains of such embodiments (where the probes are referred to as composite control probes) may range from 2 to about 10, including 2 to about 5, e.g., 2 to about 4, such as 2 to 3. Composite probes may be of interest where one wishes to use a collection of control probes made up of a minimum number of control probe domains to sample all bases at all layers of test probes that are longer than 30 mers, e.g., 45 mers, 60 mers, 75 mers, 90 mers, etc.

For example, where the test probes on a given array are 60 mers, one could have a collection of control probes made up of a single set of control probes each being 60 bases long and made up of 2 out of four possible probe domains. Such composite probes may be defined by the formula:

$$T\text{-}D_1\text{-}D_2$$

where T is an optional tether sequence, defined below, and
$D_1$ and $D_2$ are probe domains.

For example, consider 4 sequences of length $\leq 30$ (i.e., probe domains) that employ a different base at each position. Designate these sequences A, B, C and D. Then the composite probes may be described by the formulas:

    5'-A-B-T-surface
    5'-B-A-T-surface
    5'-C-D-T-surface
    5'-D-C-T-surface

The above collection of composite control probes of two different probe domains are capable of sampling all bases at all layers of these domains.

Where composite control probes are employed, any convenient protocol for identifying suitable sequences for each of the composite probe domains (and therefore complementary targets) that satisfies the above parameters may be employed. Suitable control probes may be selected, for example, by generating test control probes and testing them *in silica,* e.g., by using BLAST or any other sequence comparison program to determine if the test control probe is likely to bind to other sequences, or, for example, by generating test control probes and testing them experimentally, e.g., by performing binding assays (for example, hybridization assays) to determine if the control probe significantly binds to targets in a chosen sample. Suitable control probes may also be selected if a suitable control target has been identified: a suitable control probe will normally have a sequence that is complementary to the sequence of a suitable target.

Suitable control probes may have a known or unknown sequence, or a specific or random sequence, depending on how the control probe is selected. Control probes may have have a sequence that is not present in, and will not hybridize to, the genome of an organism represented by the test probes on a multiple-array. In other words, in certain embodiments, if a multiple-array contains test probes for genes and gene products of a specific species, each control probe on the multiple-array substrate may have a sequence that is not represented in the genome of that species or its gene products. For example, in an embodiment where the test probes are derived from a human, control probes may be from yeast, bacteria or any other organism, or may have any other sequence, including a synthetic sequence that is not found or known to be found in any organism, such that they will not specifically bind to targets in a sample from humans.

In certain embodiments, the control probes contain a tether (i.e.,linker) sequence between the first nucleotide of the control probe, i.e., the nucleotide in the control probe that is closest to the substrate, and the substrate of the array. In some embodiments, this tether sequence ranges from about 1 to about 25 , residues, e.g., from about 2 to about 15 residues, including from about 3 to about 10 residues, such as about 4, about 5, about 6 or 7 residues, where the sequence may be random and any convenient sequence, where the sequence may be homogeneous or heterogeneous with respect to the nature of the bases that make up the sequence. A representative tether domain is sequence of 5 T residues. An example of a set of control probes each with two probe domains and a 5 T linker sequence as described above is provided in Table 1.

### Table 1

| Name | Sequence |
|------|----------|
| EQP1_Pro25G_T5 | GGTCAGTGTATCCAGTT*TC*TCCGAAATCATCGTAGCTGGTCAG*TG*TATCCTTTTT (SEQ ID NO:01) |
| Pro25G_EQP1_T5 | ATCATCGTAGCTGGTCAG*TG*TATCCGGTCAGTGTATCCAGTT*TC*TCCGAATTTTT (SEQ ID NO:02) |
| EQCP2_EQCP3_T5 | TCAGGTCAGTGATTCA*CC*GAATCTGCAGTCAACCCAGACAG*GA*ACGGAGTTTTT (SEQ ID NO:03) |
| EQCP3_EQCP2_T5 | CAGTCAACCCAGACAG*GA*ACGGAGTCAGGTCAGTGATTCAC*CG*AATCTGTTTTT (SEQ ID NO:04) |

In certain embodiments, the set of probes that is employed is a set that includes a defined or known deletion in each domain relative to a full-length set, as illustrated above, such that one has a collection of "deletion" probes. A representative embodiment of such a set would be a set of control probes that includes a set of probes having the same sequence as the probes appearing in Table 1, but for the absence of the bolded and italicized base residues, making the second sub-set a deletion set relative to the first set, where the deletion is defined and known to be at residue 7 of each probe domain of the set. In certain embodiments, the set of control probes may include two or more subsets, with a first set comprising full-length sequences and the one or more subsets being defined variants of the first set, as illustrated above.

In certain embodiments, control probes may be present at any position on the sub-arrays of a multiple-array substrate. As such, the individual control probes of a set may each be present at different positions of a sub-array, e.g., random or pre-determined positions of a multiple-array substrate, or may be in close proximity to each other, e.g., in a line or row. In certain embodiments, the control probes are present at positions of a multiple-array substrate at which they are most likely to detect cross-contamination, e.g., at or near the edges of each sub-array of the multiple-array, particularly near edges of sub-arrays that are proximal to other sub-arrays of the multiple-array, where cross-contamination is more likely to occur. In certain embodiments, control probe sets are situated in each sub-array such that they will sample every synthesis nozzle in every column of synthesis at every layer of synthesis. Representative methods for determining the placement of the control probe set are provided in United States patent application serial no. 10/407,080.

*Targets*

In practicing the subject invention, a set of control target nucleic acids, e.g., labeled control target nucleic acids, corresponding to each probe domain of the control probes in the collection is employed. In certain embodiments, the control targets are designed to be complementary to one and only one of the probe domains present in the control probe set. As such, each control target is unique in sequence and binds to one and only one probe domain under conditions suitable for specific binding. In addition, the control targets should not bind to each other under conditions suitable for specific binding and, as with the control probes, control targets should not have appreciable secondary structure. Finally, it is also advantageous to design control targets such that the melting temperature of each control target:probe domain is similar. The goal in designing the control target set is to ensure that the targets of the set exhibit predictable and distinct binding characteristics to the set of control probes present in each sub-array such that the occurrence of either probe synthesis errors or cross contamination between samples contacted to each sub-array of the multiple-array can be determined (discussed below).

METHODS

In general, the methods of the subject invention include: (a) contacting a first array of a multiple-array substrate with a first sample under conditions suitable for specific binding of a first control target in the sample to a first set of at least four control probes in the first array; and (b) evaluating binding of the control target to the control probes to determine whether probe synthesis errors and/or cross-contamination between the samples has occurred. In certain embodiments, the methods at least further include contacting a second array with a second sample under conditions suitable for specific binding of a second control target to a second set of at least four control probes in the second array.

*Sample preparation*

Preparation of the samples of target nucleic acids to be contacted to each sub-array of the multiple-array substrate can be done using a variety of methods. In certain embodiments, sample preparation includes labeling of the target nucleic acids with a label, e.g., a member of signal producing system. Target samples may be labeled using any known labeling methods. Methods for labeling proteins and nucleic acids are generally well known in the art (e.g. Brumbaugh et al Proc Natl Acad Sci U S A 85, 5610-4, 1988; Hughes et al. Nat Biotechnol 19, 342-7, 2001, Eberwine et al Biotechniques. 20:584-91, 1996, Ausubel, et al, Short Protocols in Molecular Biology, 3rd ed., Wiley & Sons, 1995 Sambrook, et al, Molecular Cloning: A Laboratory Manual, Third Edition, 2001 Cold Spring Harbor, N.Y. and DeRisi et al. Science 278:680-686, 1997; Patton WF. Electrophoresis. 2000 21:1123-44; MacBeath G. Nat Genet. 2002 32 Suppl: 526-32; and Biotechnol Prog. 1997 13:649-58). These means usually involve either direct chemical' modification of the analyte, or a labeled nucleotide that is incorporated into a nucleic acid by nucleic acid replication, e.g., using a polymerase.

Chemical modification methods for labeling a nucleic acid sample usually include incorporation of a reactive nucleotide into a nucleic acid, e.g., an amine-allyl nucleotide derivative such as 5-(3-aminoallyl)-2'-deoxyuridine 5'-triphosphate, using an RNA-dependent or DNA-dependent DNA or RNA polymerase, e.g., reverse transcriptase or T7 RNA polymerase, followed by chemical conjugation of the reactive nucleotide to a label, e.g. a N-hydroxysuccinimdyl of a label such as Cy-3 or Cy5 to make a labeled nucleic acids. Such chemical conjugation methods may be combined with RNA amplification methods, to produce labeled DNA or RNA.

Suitable labels may also be incorporated into a sample by means of nucleic acid replication, where modified nucleotides such as modified deoxynucleotides, ribonucleotides, dideoxynucleotides, etc., or closely related analogues thereof, e.g. a deaza analogue thereof, in which a moiety of the nucleotide, typically the base, has been modified to be bonded to the label. Modified nucleotides are incorporated into a nucleic acid by the actions of a nucleic acid-dependent DNA or RNA polymerases, and a copy of the nucleic acid in the sample is produced that contains the label. Methods of labeling nucleic acids by a variety of methods, e.g., random priming, nick translation, RNA polymerase transcription, etc., are well generally known in the art.

Labels of interest include directly detectable and indirectly detectable radioactive or non-radioactive labels such as fluorescent dyes. Directly detectable labels are those labels that provide a directly detectable signal without interaction with one or more additional chemical agents. Examples of directly detectable labels include fluorescent labels. Indirectly detectable labels are those labels which interact with one or more additional members to provide a detectable signal. In this latter embodiment, the label is a member of a signal producing system that includes two or more chemical agents that work together to provide the detectable signal. Examples of indirectly detectable labels include biotin or digoxigenin, which can be detected by a suitable antibody coupled to a fluorochrome or enzyme, such as alkaline phosphatase. In many preferred embodiments, the label is a directly detectable label. Directly detectable labels of particular interest include fluorescent labels.

Fluorescent labels that find use in the subject invention include a fluorophore moiety. Specific fluorescent dyes of interest include: xanthene dyes, e.g. fluorescein and rhodamine dyes, such as fluorescein isothiocyanate (FITC), 6-carboxyfluorescein (commonly known by the abbreviations FAM and F),6-carboxy-2',4',7',4,7-hexachlorofluorescein (HEX), 6-carboxy-4', 5'-dichloro-2', 7'-dimethoxyfluorescein (JOE or J), N,N,N',N'-tetramethyl-6-carboxyrhodamine (TAMRA or T), 6-carboxy-X-rhodamine (ROX or R), 5-carboxyrhodamine-6G (R6G$^5$ or G$^5$), 6-carboxyrhodamine-6G (R6G$^6$ or G$^6$), and rhodamine 110; cyanine dyes, e.g. Cy3, Cy5 and Cy7 dyes; coumarins, e.g umbelliferone; benzimide dyes, e.g. Hoechst 33258; phenanthridine dyes, e.g. Texas Red; ethidium dyes; acridine dyes; carbazole dyes; phenoxazine dyes; porphyrin dyes; polymethine dyes, e.g. cyanine dyes such as Cy3, Cy5, etc; BODIPY dyes and quinoline dyes. Specific fluorophores of interest that are commonly used in subject applications include: Pyrene, Coumarin, Diethylaminocoumarin, FAM, Fluorescein Chlorotriazinyl, Fluorescein, R110, Eosin, JOE, R6G, Tetramethylrhodamine, TAMRA, Lissamine, ROX, Napthofluorescein, Texas Red, Napthofluorescein, Cy3, and Cy5, etc.

In certain embodiments of the subject invention, where the multiple-arrays include "all-bases-all-layers" control probes as described above, a collection of corresponding labeled control targets is typically included in the samples. In these embodiments, one or more labeled control targets of a control target set is usually present in each sample prior to its contact with a sub-array of a multiple array. In certain embodiments, the presence of one or more labeled control targets in a sample distinguishes that sample from all other samples to be contacted with the sub-arrays of the multiple array. In other words, the one or more labeled control targets in a sample provides a unique designation that is particular and unique to the sample as compared to the other samples for application to the other sub-arrays of the multiple-array. At least one control target thus provides a signature for the sample, the signature defined by the presence, absence or level of the at least one control target. In certain embodiments, each sample has a distinct control target signature whereas in other embodiments, a subset of samples applied to the sub-arrays of the multiple-array may have the same control target signature.

In certain embodiments, control targets are labeled independently of the rest of the targets of the sample, and are spiked (e.g., added or mixed) into the rest of the sample prior to contacting the sample to the desired array. For example, control targets may be labeled using a T7 RNA amplification labeling procedure and stored, each labeled control target in a separate tube. As needed, a desired quantity of a labeled control target is usually aliquoted from the storage tube into a sample tube and mixed with the analyte sample prior to application of the sample onto an array. Control targets may be added to a tube prior to, at the same time as, or after the addition of an analyte sample to a tube.

In certain embodiments, one labeled control target of the control target set is spiked into each sample prior to contacting the sample to the desired sub-array. For example, when using a 4-plex array, 4 distinct control targets will be placed individually into the 4 samples being applied to the sub-arrays such that control target-1 is spiked into the sample that is contacted to sub-array 1, control target-2 is spiked into the sample that is contacted to sub-array 2, and so on. In some of these embodiments, all of the control targets of the control target set are labeled with the same label. In these cases, each sample is distinguished by the identity of the specific control target spiked into it. In other embodiments, each control target can be labeled with a distinguishing label such that each sample is distinguished from all other samples both by the identity of the control target and by the label used to label each control target.

In certain embodiments, the specific control target spiked into each sample may be identical for some or all of the samples applied to each sub-array of a multiple-array substrate. In some of these embodiments, the control target to be spiked into each sample is labeled with the same label, e.g., where the control targest are spiked into different samples and the different samples are applied to different subarrays. In some of these embodiments, the control target to be spiked into each sample is labeled such that it is distinguishable from the identical targets spiked into other samples. As such, the control targets present in the samples to be applied to a sub-array of a multiple-array may, collectively, be labeled with 2, 3, 4, 5, 6, 7 or 8 or more, up to about 10, 12 or 14 or more, sometimes up to about 20 distinguishable labels. In one example, each sample to be applied to a 4-plex multiple array is spiked with labeled control targets such that in samples 1-4 to be contacted to sub-arrays 1-4, respectively, sample 1 contains control target 1 labeled with Cy3, sample 2 contains control target 1 labeled with Cy5, sample 3 contains control target 2 labeled with Cy3, and sample 4 contains target 2 labeled with Cy5.

In certain embodiments, more than one labeled control target is spiked into each sample. As such, each sample contacted to a multiple-array can contain 2 or more labeled control targets, including 2 control targets, 3 control targets, or 4 or more control targets, including 5, 6,7, 8, up to 10 or more control targets. In some of these embodiments, each sample contacted to the sub-arrays of a multiple-array substrate may contain the same number of labeled control targets whereas in other embodiments some samples contain different numbers of labeled control targets. Furthermore, the control targets spiked into a sample may be labeled with the same label or with distinguishing labels.

In certain embodiments, the labels used in the subject methods are distinguishable, meaning that the labels can be independently detected and measured, even when the labels are mixed. In other words, the amounts of label present (e.g., the amount of fluorescence) for each of the labels are separately determinable, even when the labels are co-located (e.g., in the same tube or in the same duplex molecule or in the same feature of an array). Suitable distinguishable fluorescent label pairs useful in the subject methods include Cy-3 and Cy-5 (Amersham Inc., Piscataway, NJ), Quasar 570 and Quasar 670 (Biosearch Technology, Novato CA), Alexafluor555 and Alexafluor647 (Molecular Probes, Eugene, OR), BODIPY V-1002 and BODIPY V1005 (Molecular Probes, Eugene, OR), POPO-3 and TOTO-3 (Molecular Probes, Eugene, OR), and POPRO3 and TOPRO3 (Molecular Probes, Eugene, OR). Further suitable distinguishable detectable labels may be found in Kricka et al. (Ann Clin Biochem. 39:114-29, 2002).

In making a labeled control target, it is generally desirable to label the target in a single reaction tube, and then add a portion of the labeled control target to the appropriate samples containing the test targets prior to their incubation with arrays.

*Contact and Wash*

Following sample preparation, the sample is contacted with the array under hybridization conditions, e.g., stringent hybridization conditions. In certain embodiments, a sample containing a labeled control target is contacted with a sub-array of a multiple-array substrate by transferring, e.g., pipetting, sample from a sample tube directly onto the surface of a sub-array, or, in alternative embodiments, onto a sub-array cover (such as a plastic film or coverslip) that is placed on the array, sample side towards the sub-array. In some embodiments, this process is repeated for each sub-array of a multiple-array substrate until a plurality of samples, which may be the same or different, are contacted with the sub-arrays such that one sample is independently contacted with each sub-array.

Conditions for the incubation, or hybridization, step are stringent as described above. The contacting period allows for the test and control targets in the sample to bind to the test and control probes on the array in a sequence-specific manner, i.e., targets in the sample bind to probes containing complementary sequences. In certain embodiments, after the contact/incubation step, the arrays are washed to remove test and control targets that are not bound to their corresponding test or control probes.

*Reading the Array*

After the contacting and wash steps, reading of the array may be accomplished by illuminating the array and reading the location and intensity of resulting fluorescence at each feature of the array to detect any binding complexes on the surface of the array. For example, a scanner may be used for this purpose, such as the AGILENT MICROARRAY SCANNER device available from Agilent Technologies, Palo Alto, CA. Other suitable scanner devices and methods are described in U.S. Patent Nos. 5,091,652; 5,260,578; 5,296,700; 5,324,633; 5,585,639; 5,760,951; 5,763,870; 6,084,991; 6,222,664; 6,284,465; 6,371,370 6,320,196 and 6,355,934. However, arrays may be read by any other method or apparatus than the foregoing, with other reading methods including other optical techniques (for example, detecting chemiluminescent or electroluminescent labels) or electrical techniques (where each feature is provided with an electrode to detect hybridization at that feature in a manner disclosed in US 6,221,583 and elsewhere). Results from the reading may be raw results (such as fluorescence intensity readings for each feature in one or more color channels) or may be processed results such as obtained by rejecting a reading for a feature which is below a predetermined threshold and/or forming conclusions based on the pattern read from the array (such as whether or not a particular target sequence may have been present in the sample). The results of the reading (processed or not) may be forwarded (such as by communication) to a remote location if desired, and received there for further use (such as further processing).

After incubation of the array under conditions suitable for specific binding of the targets to the probes, the level of binding of the control targets to the control probes is evaluated. If the observed binding characteristics of the control targets in each sample to the control probe set on each sub-array correspond to the predicted binding characteristics, then no probe synthesis errors or cross-contamination between samples has occurred. However, if the observed binding characteristics of the control targets in each sample to the control probe sets on each sub-array do not correspond to the predicted binding characteristics, probe synthesis errors and/or cross-contamination between samples may have occurred. Binding characteristics can include the pattern of target-bound probe on the array, e.g., identifying which probes are bound to a labeled target (e.g., the level of label detected at a specific probe location is above the background level), and the quantity of labeled target bound to a probe (e.g., comparing the level of label detected at a specific probe location to one or a number of internal or independently generated controls).

*Identifying Cross contamination*

For a single array, the control target in the sample applied to the sub-array binds to the corresponding control probe containing the probe domain that is complementary to it. However, when cross-contamination occurs, a control target that was not intended to be present in the sample at the time it was contacted to the sub-array binds to its corresponding probe domain in the control probe set on the sub-array. When the sub-array is read, binding of the cross-contaminating target to its control probe (or probes) containing the corresponding probe domain will be detected. As such, the pattern of control probe binding in a cross-contaminated sample will not correspond to the pattern expected of the intended control target present in the sample contacted to the sub-array. In other words, if the control targets in the samples to be applied to the arrays of a multi-array substrate are labeled, cross-contamination is detected if, within a sub-array, label is detected on control probes that do not correspond to the control target intended to be present in the sample contacted to that sub-array. As such, an unexpected control probe set binding pattern indicates the presence of contamination between samples applied to the sub-arrays of a multi-subunit array.

Examples of causes of cross-contamination may include: errors during transfer of sample from a tube onto an array (e.g., pipetting a sample that is intended to be contacted with one array onto two arrays, or pipetting two samples consecutively without changing or cleaning the pipette dispenser used for transfer), sample leakage from one sub-array of a multi-array substrate to another sub-array of a multi-array substrate after contacting the sub-arrays with samples and during incubation under specific binding conditions, and contamination that occurs during washing of the substrate. It is also possible to determine whether samples were switched after control target spiking. For example, if the control probe binding pattern in sub-array 1 appears identical to that expected in sub-array 2 and vice versa, the samples may be determined to have been switched prior to the contacting step.

In certain embodiments, the amount of contamination can be estimated by comparing the levels of control target binding on all control probes in the set, both expected and unexpected. In other words, if a control probe:control target binding pattern indicates that cross-contamination has occurred, the binding pattern may be employed to indicate the source and level of contamination. For example, if a control probe on the first sub-array of a multiple-array substrate is bound by a control target that was only added to a sample applied to the second sub-array, then binding to that probe indicates that cross-contamination has occurred, and that the contaminating sample is the sample applied to the second sub-array of the substrate. Furthermore, by quantifying, at least relatively, the amount of the cross-contaminating control target in the contaminating sample to the control probes on the first and second sub-arrays, the level of cross contamination may be estimated. For example, if the level of binding of the cross-contaminating control target and the intended control target (e.g., the control target that was intentionally spiked into the sample) to the corresponding control probe is similar, the amount of cross-contamination is likely to be high. However, if binding of a cross-contaminating control target to a corresponding control probe is barely statistically significant, the amount of cross-contamination is likely to be low.

In other embodiments, particularly those in which control targets are labeled with distinguishable labels to provide a distinguishing difference between each of the samples to be applied to sub-arrays of a multiple-array, no cross-contamination is detected if binding of the control probes to the control targets is as expected, e.g., a single distinguishable label is associated with each of the corresponding control probes on each of the sub-arrays of the multiple-array substrate. If this is not the case, for example, if a control probe on one or more of the sub-arrays of the multiple-array substrate is associated with more than one distinguishable label (e.g., two distinguishable labels), cross contamination may have occurred. In a representative embodiment, a control target is labeled with Cy3 and added to a first sample, and the same control target is labeled with Cy5 and added to a second sample. The first sample is applied to a first sub-array of a multiple-array substrate and the second sample is added to a second sub-array of the multiple-array substrate and incubated under conditions sufficient for binding of the probes to the targets. After washing, the binding patterns of the control targets to the corresponding control probes present on both sub-arrays are detected. If the signal detected with the corresponding control probe on the first sub-array is a Cy3 signal and the signal detected with the corresponding control probe on the second sub-array is a Cy5 signal, then no cross-contamination has occurred. If either of the signals does not entirely correspond to a signal from only Cy3 or Cy5 (e.g., the signal detected is a mixture of Cy3 *and* Cy5), then cross-contamination may have occurred.

The following description references the exemplary embodiment illustrated in Figure 1. It is not intended that the invention should be limited to the embodiments shown in this figure. Upon description of the embodiments illustrated in Figure 1, other embodiments that are not specifically described in the figure will become apparent to one of skill in the art.

Figure 1 shows a representative 8 pack multiple-array substrate containing the 4 composite control probes shown in Table 1. In this example, the control probe set is printed in sub-arrays 1-1 to 2-4 in every nozzle synthesis column. The probe domain structure of the control probes in Fig.2 is as follows (each domain is represented A, B, C, D):

    Control Probe 1: A-B-(TTTTT)-substrate
    Control Probe 2: B-A-(TTTTT)-substrate
    Control Probe 3: C-D-(TTTTT)-substrate
    Control Probe 4: D-C-(TTTTT)-substrate

The samples applied to sub-arrays 1-1, 1-2, 1-3 and 1-4 contain Cy3-labeled control targets TAR25C (binds to probe domain A), EQCT1 (binds to probe domain B), EQCT2 (binds to probe domain C) and EQCT3 (binds to probe domain D), respectively. In this configuration, when no cross-contamination occurs, control probes 1 and 2 in sub-arrays 1-1 and 1-2 show detectible levels of binding whereas probes 3 and 4 do not, and the reverse is true for sub arrays 1-3 and 1-4. However, if the sample contacted to sub-array 1-2 is contaminated with the' sample contacted to sub-array 1-3, probes 3 and 4 in addition to probes 1 and 2 in sub-array 1-2 would have detectible levels of binding. In this example, cross-contamination between the samples contacted to sub-arrays 1-1 and 1-2 as well as between the samples contacted to sub arrays 1-3 and 1-4 would be difficult to detect. In an alternative embodiment, if control target EQCT1 and EQCT3 were labeled with Cy5 instead of Cy3, all possible combinations of cross-contamination could be detected. In this embodiment, if the sample contacted to sub-array 1-1 is contaminated with the sample contacted to sub-array 1-2, then control probes 1 and 2 in sub-array 1-1 would have detectible Cy3 *and* Cy5 label, whereas in the absence of cross contamination, control probes 1 and 2 in sub array 1-1 will have only detectable Cy3 label.

Alternatively, if the composite control probes have a different configuration, the control targets can all be labeled with Cy3 and still detect all possible cross-contaminations between samples applied to sub-arrays 1-1 to 1-4. Specifically, if the domain structure were changed to the following:

    Control Probe 1': A-B-(TTTTT)-substrate
    Control Probe 2': B-C-(TTTTT)-substrate
    Control Probe 3': C-D-(TTTTT)-substrate
    Control Probe 4': D-A-(TTTTT)-substrate

then all combinations of cross contamination between the samples contacted to each sub-array can be detected. For example, in the original configuration, cross-contamination between samples contacted to sub-arrays 1-1 and 1-2 may be difficult to detect because both targets bind to the same control probe, i.e., 1 and 2. However, using control probes with the alternative configuration (1' - 4'), cross contamination of the sample contacted to sub-array 1-1 with the sample contacted to sub-array 1-2 is easily detectible because the pattern of control probe binding will be different. Specifically, when no contamination occurs, only control probes 1' and 4' have detectible label in sub-array 1-1 whereas when contamination with the sample contacted to sub-array 1-2 occurs, control probe 2', in addition to control probes 1' and 4', will also have detectible label.

Methods of using spike-in probes for identification of cross-contamination, which methods are readily adapted to methods of the subject invention, are further described in United States Application Serial No. 10/655,430 (Agilent Docket No. 10030054-1, filed September 3, 2004).

*Identifying Probe Synthesis Errors*

In addition to, or instead of, detection of cross-contamination between samples contacted to different sub-arrays of a multiple-array, the sets of control probes and control targets of the subject invention can also detect synthesis errors thus providing an added layer of quality control in the use of multiple-arrays. In certain embodiments, the placement and domain structure of the set of control probes of the subject invention in each sub-array of a multiple array is such that the function of every probe synthesis nozzle can be determined at every layer of synthesis during the array fabrication process. Synthesis errors can result in the production of probes that are truncated or that are missing specific bases at specific layers of synthesis. These errors change the level of complimentary of the probe to its specific target and thus can alter the level of target binding. Without a measure of probe synthesis accuracy, this reduction in probe:target interaction may lead to a disconnect between the actual level of a specific target present in a sample and the measured level.

In the example shown in Figure 1, each control probe in the set of 4 control probes contains 2 domains chosen from a set of 4 distinct probe domains ("all-bases-all-layers" probe domains). In this set of control probes, each domain is present in both "early" and "late" positions with respect to probe synthesis. In this example, every base is sampled at every layer of synthesis for every nozzle position because the set of 4 control probes is printed in every column of probe synthesis. When no probe synthesis errors have occurred, the level of detectible target binding to each specific probe in the set is equivalent, i.e., the same level. In sub-array 1-1, for example, the level of detectable binding of TAR25C-Cy3 to control probes 1 and 2, both of which contain its complimentary probe domain, will be the same when no synthesis errors have taken place. However, if a probe synthesis error occurred during the fabrication process, the specific control probes in the column with the defective nozzle will have an error which reduces its affinity to its corresponding control target. Depending on when the nozzle failure took place, the error will either be in domain 1 (late during the synthesis process) or domain 2 (early in the synthesis process). For example, if a probe synthesis error took place early during synthesis in sub-array 1-1, the binding of TAR25C-Cy3 to probe 2 in the affected nozzle column would be less that that detected to probe 1. Because each sample contacted to each sub-array of the multiple-array contains a labeled control target that is complementary to a probe domain that is present in the set of control probes in both early and late synthesis positions, early and late synthesis errors can be detected in all sub-arrays

In the example shown in Figure 1, both probe synthesis and cross-contamination errors can be detected simultaneously. As mentioned above, if the sample contacted to sub-array 1-2 is contaminated with sample contacted to sub-array 1-3, then in addition to the expected detection of target binding to control probes 1 and 2, target binding will also be detected on control probes 3 and 4, the level of which may indicate the level of cross-contamination. Furthermore, if an early probe synthesis error occurred in one of the synthesis columns in sub-array 1-2, the specific probe in that column would have reduced binding, i.e., control probe 1 would have less detectible binding than control probe 2 in the affected column.

The alternative probe configuration described above in the cross-contamination section (i.e., probes 1' - 4') will also work for detecting probe synthesis errors because each control probe domain is present in both early and late synthesis positions. In this example, an early probe synthesis error occurring in sub array 1-1 would result in a reduced detectible binding of the spiked control target, i.e., TAR25C, to probe 4' as compared to probe 1'. Similar control probe binding analysis would allow for the detection of other probe synthesis errors.

Detection of probe synthesis errors using "all-bases-all-layers" control probe sets if further described in United States patent application serial no. 10/407,090.

UTILITY OF ARRAYS

The subject arrays find use in a variety applications, where such applications are generally analyte detection applications in which the presence of a particular analyte in a given sample is detected at least qualitatively, if not quantitatively. Protocols for carrying out such assays are well known to those of skill in the art and need not be described in great detail here. Generally, the sample suspected of comprising the analyte of interest is contacted with an array produced according to the subject methods under conditions sufficient for the analyte to bind to its respective binding pair member that is present on the array. Thus, if the analyte of interest is present in the sample, it binds to the array at the site of its complementary binding member and a complex is formed on the array surface. The presence of this binding complex on the array surface is then detected, e.g. through use of a signal production system, e.g., an isotopic or fluorescent label present on the analyte, etc. The presence of the analyte in the sample is then deduced from the detection of binding complexes on the substrate surface.

Specific analyte detection applications of interest include hybridization assays in which the nucleic acid arrays of the subject invention are employed. In these assays, a sample of target nucleic acids is first prepared, where preparation may include labeling of the target nucleic acids with a label, e.g., a member of signal producing system. Where the arrays include "all-bases-all-layers" control probes, as described above, a collection of labeled control targets may be included in the sample, where the collection may be made up of control targets that are all labeled with the same label or two or more sets that are distinguishably labeled with different labels, as described above.

Specific hybridization assays of interest which may be practiced using the subject arrays include: gene discovery assays, differential gene expression analysis assays; nucleic acid sequencing assays, and the like. Patents and patent applications describing methods of using arrays in various applications include: 5,143,854; 5,288,644; 5,324,633; 5,432,049; 5,470,710; 5,492,806; 5,503,980; 5,510,270; 5,525,464; 5,547,839; 5,580,732; 5,661,028; 5,800,992.

In some embodiments, the subject methods include a step of transmitting data from at least one of the detecting and deriving steps, as described above, to a remote location. The data may be transmitted to the remote location for further evaluation and/or use. Any convenient telecommunications means may be employed for transmitting the data, e.g., facsimile, modem, internet, etc.

PROGRAMMING

The invention also provides programming for analysis of array data to determine if cross-contamination and/or probe synthesis errors have occurred. In general, once the set of control probes and targets have been defined for an array and an expected binding pattern to those arrays, in the absence of cross-contamination of probe synthesis errors, has been established, the subject programming may analyze data from the array and determine if unexpected binding of target to the control probes has occurred. For example, if cross-contamination has occurred, the programming may identify or flag data as being unreliable.

Such programming may be readily incorporated into any features extraction or any data analysis program. Several commercially available programs perform feature extraction on microarrays, such as IMAGINE ® by BioDiscovery (Marina Del Rey, CA) Stanford University's "ScanAlyze" Software package, Microarray Suite of Scanalytics (Fairfax, VA), "DeArray" (NIH); PATHWAYS ® by Research Genetics (Huntsville, Ala.); GEM tools ® by Incyte Pharmaceuticals, Inc., (Palo Alto, Calif.); Imaging Research (Amersham Pharmacia Biotech, Inc., Piscataway, N.J.); the RESOLVER ® system of Rosetta (Kirkland, WA ) and the Feature Extraction Software of Agilent Technologies (Palo Alto, CA). Such commercially available programs may be adapted or modified to perform the subject methods.

Programming according to the present invention, i.e., programming that allows one to identify cross-contamination and/or probe synthesis errors as described above, can be recorded on computer readable media, e.g. any medium that can be read and accessed directly by a computer. Such media include, but are not limited to: magnetic storage media, such as floppy discs, hard disc storage medium, and magnetic tape; optical storage media such as CD-ROM; electrical storage media such as RAM and ROM; and hybrids of these categories such as magnetic/optical storage media. One of skill in the art can readily appreciate how any of the presently known computer readable mediums can be used to create a manufacture that includes a recording of the present programming/algorithms for carrying out the above described methodology.

KITS

Kits for use in analyte detection assays are also provided. The kits at least include the arrays of the invention, as described above. Where the arrays include the above described "all-bases-all-layers" control probes, the kits may further include labeled collections of control target nucleic acids (or precursors thereof, e.g., template nucleic acids thereof) made up of targets corresponding to the probe domains present in the of the set of control probes on the array, where the collection of control targets may be labeled with the same label or two or more sets of distinguishable labels. The kits may further include one or more additional components necessary for carrying out an analyte detection assay, such as sample preparation reagents, buffers, labels, and the like. As such, the kits may include one or more containers such as vials or bottles, with each container, containing a separate component for the assay, and reagents for carrying out an array assay such as a nucleic acid hybridization assay or the like. The kits may also include a denaturation reagent for denaturing the analyte, buffers such as hybridization buffers, wash mediums, enzyme substrates, reagents for generating a labeled target sample such as a labeled target nucleic acid sample, negative and positive controls and written instructions for using the array assay devices for carrying out an array based assay. Such kits also typically include instructions for use in practicing array based assays.

The instructions of the above described kits are generally recorded on a suitable recording medium. For example, the instructions may be printed on a substrate, such as paper or plastic, etc. As such, the instructions may be present in the kits as a package insert, in the labeling of the container of the kit or components thereof (i.e. associated with the packaging or sub packaging), etc. In other embodiments, the instructions are present as an electronic storage data file present on a suitable computer readable storage medium, e.g., CD-ROM, diskette, etc, including the same medium on which the program is presented.

In yet other embodiments, the instructions are not themselves present in the kit, but means for obtaining the instructions from a remote source, e.g. via the Internet, are provided. An example of this embodiment is a kit that includes a web address where the instructions can be viewed and/or from which the instructions can be downloaded. Conversely, means may be provided for obtaining the subject programming from a remote source, such as by providing a web address. Still further, the kit may be one in which both the instructions and software are obtained or downloaded from a remote source, as in the Internet or World Wide Web. Some form of access security or identification protocol may be used to limit access to those entitled to use the subject invention. As with the instructions, the means for obtaining the instructions and/or programming is generally recorded on a suitable recording medium.

Although the foregoing invention has been described in some detail by way of illustration and example for purposes of clarity of understanding, it is readily apparent to those of ordinary skill in the art in light of the teachings of this invention that certain changes and modifications may be made thereto without departing from the scope of the appended claims.

The disclosures in United States patent application No. 10/957,062, from which this application claims priority, and in the abstract accompanying this application are incorporated herein by reference.

**Claims**

1. A multiple array substrate comprising at least a first sub-array and a second sub-array, wherein each of said first and second sub-arrays comprises a set of at least four control probe nucleic acids each having at least a first probe domain of from about 4 to about 30 nucleic acid residues, wherein each member probe nucleic acid of said set has a different base from any other member of said set at each residue position of said first probe domain.

2. A multiple array substrate as claimed in Claim 1, wherein said multiple array substrate comprises at least four sub-arrays, each comprising said set of at least four control probes.

3. A multiple array substrate as claimed in Claim 1 or 2, wherein each member probe nucleic acid of said set further comprises a second probe domain of about 4 to about 30 nucleotide residues, wherein each member probe nucleic acid of said set has a different base from any other member of said set at each residue position of said second probe domain.

4. A multiple array substrate as claimed in Claim 3, wherein each second probe domain of said set has a sequence that is identical to the sequence of a first probe domain of said set, where the first and second domains of a given probe are not identical.

5. A multiple array substrate as claimed in any of Claims 1 to 4, wherein said set of at least four control probe nucleic acids each have the structure:

$$T\text{-}D_1\text{-}D_2$$

where:

    T is an optional tether sequence;
    D1 is a first probe domain; and
    D2 is a second probe domain.

6. A multiple array substrate as claimed in Claim 5, wherein each first probe domain D1 has a sequence chosen from the group consisting of A, B, C and D,
wherein each of A, B, C and D has a unique sequence of from 4 to 30 nt in length.

7. A multiple array substrate as claimed in Claim 6, wherein said set of at least four control probe nucleic acids is described by the formula:

$$T\text{-}A\text{-}B$$

$$T\text{-}B\text{-}A$$

$$T\text{-}C\text{-}D$$

$$T\text{-}D\text{-}C$$

8. A multiple array substrate as claimed in Claim 7, wherein A, B, C and D have the following formulas:

$$A = GGTCAGTGTATCCAGTTTCTCCGAA \text{ (SEQ ID NO: 05);}$$

$$B = ATCATCGTAGCTGGTCAGTGTATCC \text{ (SEQ ID NO: 06);}$$

C = TCAGGTCAGTGATTCACCGAATCTG (SEQ ID NO: 07);

and

D = CAGTCAACCCAGACAGGAACGGAGT (SEQ ID NO: 08).

9. A method comprising:

(a) contacting:

(i) a first sample with a first sub-array of a multiple array substrate according to any of Claims 1 to 8, wherein said first sample comprises a first control target nucleic acid; and
(ii) a second sample with a second sub-array of said multiple array substrate;

wherein each of said first and second sub-arrays comprises a set of at least four control probe nucleic acids each having at least a first probe domain of from about 4 to about 30 nucleic acid residues, wherein each member probe nucleic acid of said set has a different base from any other member of said set at each residue position of said first probe domain; and
(b) detecting binding of said first control target nucleic acid to said first set of control probes in each of said first and second sub-arrays.

10. A method as claimed in Claim 9, wherein said method comprises screening for the presence of a probe synthesis error.

11. A method as claimed in Claim 9 or 10, wherein said method comprises screening for the presence of cross-contamination.

12. A method as claimed in any of Claims 9, 10 or 11, wherein said method further comprises including a second control target nucleic in said second sample, wherein said second control target nucleic acid has a sequence that is different from said first control target nucleic acid and is complementary to a first probe domain of a second probe nucleic acid of said set.

13. A kit comprising:

(a) an array according to any of Claims 1 to 8; and
(b) a first control target nucleic acid that is complementary to a first probe domain of a first probe nucleic acid of said set.

14. A kit as claimed in Claim 13, wherein said kit further comprises a second control target nucleic having a sequence that is different from said first control target nucleic acid and is complementary to a first probe domain of a second probe nucleic acid of said set.

FIG. 1

SEQUENCE LISTING

<110> Agilent Technologies, Inc.

<120> Multiple Array Substrates

<130> IT/KE/N17485

<150> US10/957,062
<151> 2004-10-01

<160> 8

<170> PatentIn version 3.3

<210> 1
<211> 55
<212> DNA
<213> Artificial

<220>
<223> probe

<400> 1
ggtcagtgta tccagtttct ccgaaatcat cgtagctggt cagtgtatcc ttttt          55


<210> 2
<211> 55
<212> DNA
<213> Artificial

<220>
<223> probe

<400> 2
atcatcgtag ctggtcagtg tatccggtca gtgtatccag tttctccgaa ttttt          55


<210> 3
<211> 54
<212> DNA
<213> Artificial

<220>
<223> probe

<400> 3
tcaggtcagt gattcaccga atctgcagtc aacccagaca ggaacggagt tttt          54


<210> 4
<211> 54
<212> DNA
<213> Artificial

<220>

```
<223>  probe

<400>  4
cagtcaaccc agacaggaac ggagtcaggt cagtgattca ccgaatctgt tttt          54


<210>  5
<211>  25
<212>  DNA
<213>  Artificial

<220>
<223>  probe domain

<400>  5
ggtcagtgta tccagtttct ccgaa                                          25


<210>  6
<211>  25
<212>  DNA
<213>  Artificial

<220>
<223>  probe domain

<400>  6
atcatcgtag ctggtcagtg tatcc                                          25


<210>  7
<211>  25
<212>  DNA
<213>  Artificial

<220>
<223>  probe domain

<400>  7
tcaggtcagt gattcaccga atctg                                          25


<210>  8
<211>  25
<212>  DNA
<213>  Artificial

<220>
<223>  probe domain

<400>  8
cagtcaaccc agacaggaac ggagt                                          25
```